# EUROPEAN PATENT APPLICATION

(11) **EP 2 465 450 A1**
(43) Date of publication of application: **20.06.2012**
(21) Application number: 11194231.4
(22) Date of filing: 19.12.2011
(51) Int. Cl.: A61B 17/34

(54) **Self Deploying Bodily Opening Protector**

(30) Priority: 20.12.2010 US 201013424927; 01.09.2011 US 201113223678
(71) Applicant: Tyco Healthcare Group LP, Mansfield, MA 02048 (US)
(72) Inventor: Okoniewski, Greg, North Haven, CT Connecticut 06473 (US); Ebner, Timothy D., New Haven, CT Connecticut 06511 (US)
(74) Representative: Soames, Candida Jane

(57) **Abstract**

A self-deploying opening protection device is disclosed. The opening protection device is transitionable between compressed and expanded conditions. The opening protection device is translatable through a lumen of a cannula placed within an opening in the skin of a patient. The opening protection device deploys to the expanded condition as it translates out from within the lumen of the cannula.

## Description

### CROSS REFERENCE TO RELATED APPLICATION

The present application paragraphs the benefit of and priority to U. S. Provisional Application Serial No. 61/424,927 filed on December 20, 2010, the entire contents of which are incorporated herein by reference.

### BACKGROUND

### 1. Technical Field

The present disclosure relates to a surgical device for protecting a bodily opening, e.g., a wound or naturally occurring orifice, through which a surgical procedure is performed. More particularly, a wound protector for use during a minimally invasive surgical procedure is disclosed.

### 2. Background of Related Art

Minimally invasive surgery, e.g., laparoscopic, endoscopic, and thoroscopic surgery, has many advantages over traditional open surgeries. In particular, minimally invasive surgery eliminates the need for a large incision, thereby reducing discomfort, recovery time, and many of the deleterious side effects associated with traditional open surgery.

The minimally invasive surgeries are performed through small openings in a patient's skin. These openings may be incisions in the skin or may be naturally occurring body orifices (e.g., mouth, anus, or vagina).

When an incision is required, the initial puncture is usually very small so that a needle or trocar can achieve the desired penetration without excessive damage to nearby tissue. It may be necessary for the initial access hole to be subsequently enlarged to provide a working diameter to permit introduction of surgical instruments and the performance of the desired medical procedure.

As with any incision, the creation of a wound presents a risk of contamination of the wound, e.g., a bacterial infection. It may therefore be desirable to protect the wound edges with a wound protector to inhibit contamination of the wound. In addition, during a surgical procedure, it may be desirable to protect the inner surfaces of a bodily opening to inhibit damage and/or minimize discomfort. A continuing need exists for improved bodily opening protection devices.

### SUMMARY

Disclosed herein is an opening protection device including a liner member. The liner member is transitionable between a compressed condition and an expanded condition. The liner member defines a first width in the compressed condition and a second width in the expanded condition. The second width in the expanded condition is greater than the first width in the compressed condition. The liner member may be biased toward the expanded condition. The opening protection device also includes a cannula that is insertable into an opening, e.g., a wound, in the skin of a patient. The liner member is translatable through a lumen of the cannula. The lumen of the cannula has a dimension corresponding to the first width of the liner member while in the compressed condition. Translation of the liner member out from within the cannula effects transitioning, e.g., deployment, of the liner member to the expanded condition.

The opening protection device may also include an obturator that engages the liner member to effect translation of the liner member through the cannula. The cannula and the obturator may include corresponding threading such that rotational movement of the cannula relative to the obturator in a first direction effects distal translation of the obturator through the cannula, and rotational movement of the cannula relative to the obturator in a second direction effects proximal translation of the obturator through the cannula.

The liner member may include a proximal section including a proximal flange, and distal section including a distal flange. Disposed between the proximal and distal flanges is an intermediate section. The intermediate section is configured and adapted to line the inner surface of an opening, e.g., a natural bodily opening or a wound, within tissue.

In an embodiment, the liner member may include a proximal ring, a distal ring, and lining disposed therebetween. The proximal and distal rings may be transitionable between compressed and expanded conditions, defining first and second diameters in the compressed and expanded conditions, respectively. The diameters of the proximal and distal rings, when in the expanded condition, may be substantially the same or they may be different. The second diameter defined by the proximal and distal rings while in the expanded condition is greater than the first diameter in the compressed condition. Moreover, in an embodiment, length of the liner member may be adjustable. For example, the lining may be rolled about the proximal ring such that relative longitudinal movement of the proximal and distal rings away from one another effects a lengthening of the liner member. Conversely, relative longitudinal movement of the proximal and distal rings towards each other effects a shortening of the liner member.

The obturator may include one or more grooves to engage at least one of the proximal and distal flanges (or rings). For example, the obturator may be configured and adapted to be placed within a longitudinally extending lumen of the liner member. The one or more grooves engage the proximal and/or distal flange (or ring) such that longitudinal translation of the obturator through the cannula effects a corresponding longitudinal translation of the liner member. As the obturator distally translates the liner member out from within the lumen of the cannula, the liner member deploys to the expanded condition. Thereafter, the cannula and obturator may be removed from the opening within the patient's skin leaving the liner member deployed within the opening of the skin. When the liner is placed within the lumen of the cannula, the liner is maintained in the compressed condition by the lumen of the cannula.

A method of deploying a liner member within a bodily opening, e.g., a wound, is also described. In particular, a surgeon provides a cannula including a self-deploying liner member placed within a lumen of the cannula. The cannula is placed within the bodily opening. Thereafter, the liner member is distally translated through the cannula to effect ejection of the liner member from within the cannula and into the bodily opening. The cannula is proximally translated out from the bodily opening. The proximal translation of the cannula out form the bodily opening and the distal translation of the liner member may occur simultaneously, thereby effecting removal of the cannula and deployment of the liner member within the bodily opening in substantially one action by the surgeon.

These and other embodiments of the present disclosure will be described in detail below with reference to the accompanying figures.

### BRIEF DESCRIPTION OF THE DRAWINGS

Embodiments of the present disclosure are described herein with reference to the accompanying drawings, wherein:

Fig. 1 is a partially cut-away view of an opening protection device in accordance with the present disclosure;

Fig. 2 is a perspective view of a liner member in accordance with the present disclosure and shown in an initial, expanded condition;

Fig. 3 is the liner member of Fig. 2 shown in a second, compressed condition;

Fig. 4 is a perspective view of an obturator in accordance with the present disclosure;

Fig. 5 is a partially cut-away view of the opening protection device of Fig. 1 shown placed within a wound of a tissue in a first condition;

Fig. 6 is a partially cut-away view of the opening protection device of Fig. 1 shown placed within a wound of a tissue in an intermediate condition;

Fig. 7 is a perspective view of the opening protection device of Fig. 1 shown placed within a wound of a tissue in a deployed condition; and

Fig. 8 is another embodiment of a liner member in accordance with the present disclosure.

### DETAILED DESCRIPTION

Embodiments of the present disclosure will now be described in detail with reference to the drawings, wherein the reference numerals identify similar or identical elements. In the drawings and in the description that follows, the term "proximal," will refer to the end of a gauge or device that is closest to the operator, while the term "distal" will refer to end of the gauge or device that is farthest from the operator.

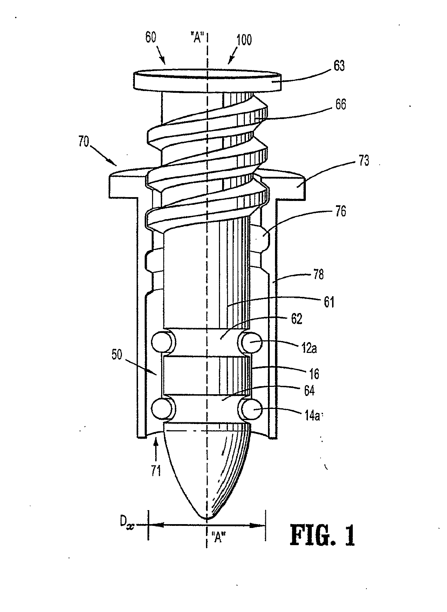

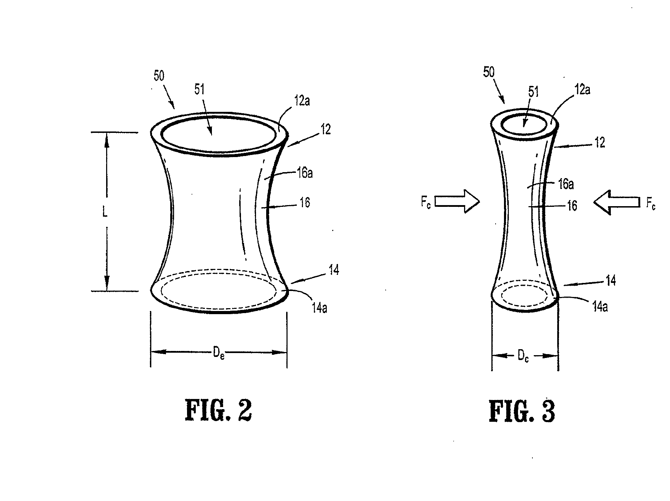

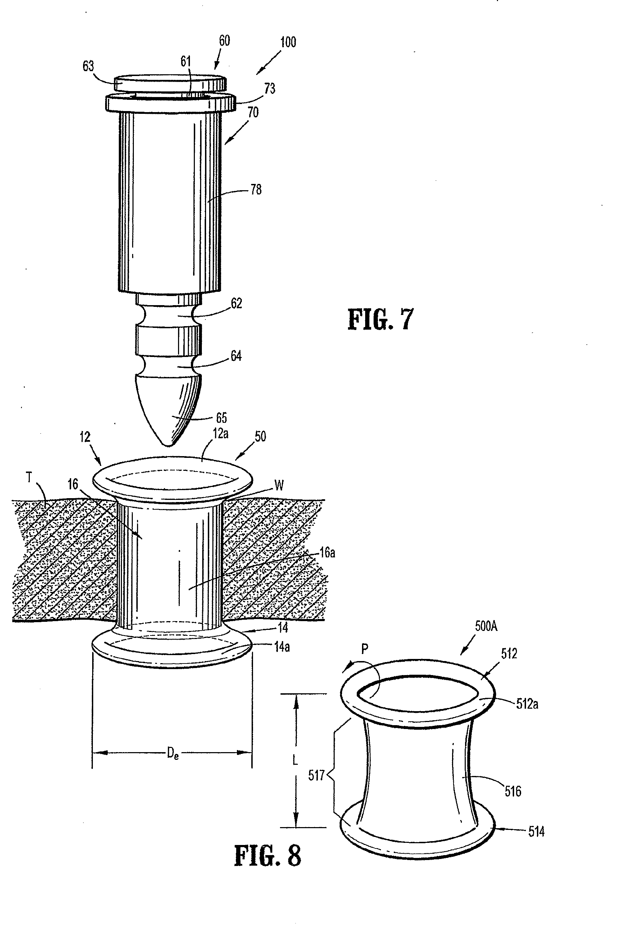

An opening protection device 100 will now be described with reference to Figs. 1-4. The opening protection device 100 includes a self-deploying liner member 50 and a cannula 70. As shown in Fig. 1, the liner member 50 is positioned within a lumen 71 of the cannula 70. As shown best in Figs. 2 and 3, the liner member 50 includes a proximal section 12, a distal section 14, and an intermediate section 16 disposed between the proximal and distal sections 12, 14. The proximal section 12 may include a proximal flange member 12a. The distal section 14 may include a distal flange member 14a. The proximal and distal flange members 12a, 14a may each have a substantially convex, e.g., curved or rounded, shape or configuration. The intermediate section 16 may include a liner 16a. In an embodiment, the liner 16a may be a thin, pliable length of material that is held taut by the proximal and distal flange members 12a, 14a when the opening protection device 100 is placed within opening W within tissue T (Fig. 7). The liner 16a defines a substantially tubular shape or configuration. In an embodiment, the liner 16a may be formed from the same or a similar material as the proximal and distal flange members 12a, 14a.

Additionally, as shown in Figs. 2, 3, and 7, the liner member 50 may have a substantially hour-glass configuration, e.g., the radial dimension defined by the intermediate section 16 may be less than the radial dimensions of the proximal section 12 and the distal section 14. Furthermore, the proximal and distal flange members 12a, 14a may have substantially the same or different radial dimensions. The hour-glass configuration of the liner member 50 may facilitate securing and/or anchoring the liner member 50 within opening W within tissue T. Moreover, the liner member 50 has a substantially tubular shape defining a longitudinally extending lumen 51.

The liner member 50 is transitionable between a first condition in which the liner member 50 defines a radial dimension having an expanded diameter De (Fig. 2), and a second condition in which the liner member 50 defines a radial dimension having a compressed diameter Dc (Fig. 3). The liner member 50 is biased toward the first condition. The liner member 50 may be formed wholly or partially from a compressive and resilient material such that the liner member 50 may be compressed by applying a compressive force Fc, and will return to a relaxed or an expanded state in the absence of the compressive force Fc.

As shown in Fig. 3, application of the compressive force Fc transitions the liner member 50 to the second condition, e.g., the radial dimension of the liner member 50 is the compressed diameter Dc. The liner member 50 is configured and adapted to be placed within a lumen 71 of the cannula 70. When the liner member 50 is within the lumen 71 of the cannula 70, the compressed diameter Dc is substantially the same as an internal diameter Dx of the cannula 70.

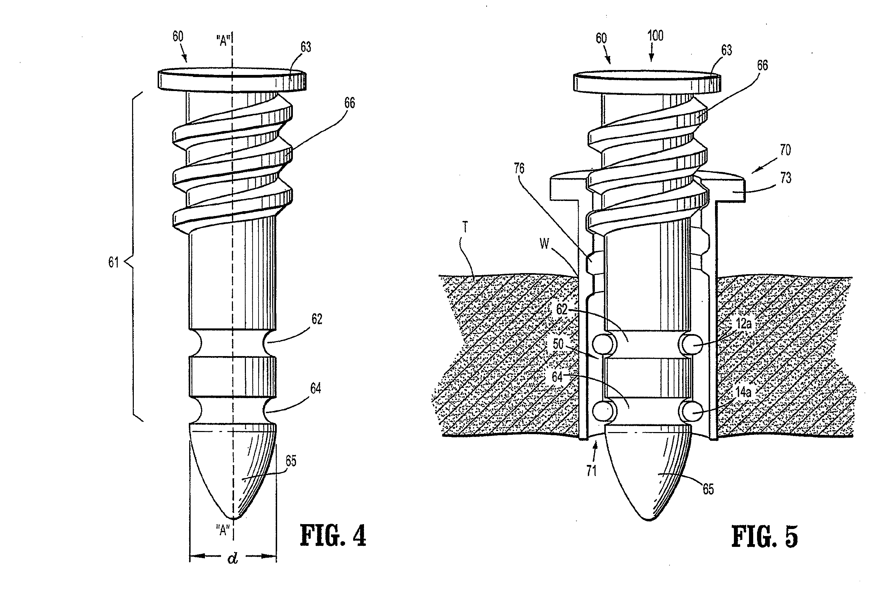

As shown in Fig. 4, an obturator 60 includes a first section 61 that is substantially cylindrical and a tapered nose 65. The first section 61 includes one or more grooves 62, 64 orthogonally disposed with respect to the longitudinal axis "A" and circumscribing the first section 61. The one or more grooves 62, 64 may be proximal to the tapered nose 65 of the obturator 60. Each groove 62, 64 is configured and adapted to releasably receive a portion of one of the proximal and distal flanges 12a, 14a of the liner member 50.

The obturator 60 may also include a lip 63 that is configured and adapted to engage lip 73 of the cannula 70. The interaction between the lip 63 of the obturator 60 and the lip 73 of the cannula 70 inhibits relative translation of the obturator 60 with respect to the cannula 70 beyond a given range of motion. Additionally, the obturator 60 may include threading 66 engageable with threading 76 of the cannula. The obturator 60 is insertable into and translatable through the lumen 71 of the cannula 70. The obturator 60 is also insertable into and translatable through the lumen 51 of the liner member 50. The tapered noses 65 of the obturator may facilitate insertion of the obturator through the liner member 50 and the cannula 70, as well as insertion of the opening protection device 100 into the opening W of the tissue T.

In an embodiment, as shown in Fig. 8, a liner member 500A is substantially similar to liner member 50 with the following differences. The liner member 500A includes a proximal end 512, a distal end 514, and an intermediate section 517 disposed between the proximal and distal ends 512, 514. A length of lining 516 is operably coupled or attached to the distal flange 514. A proximal ring 512a is configured and adapted to receive the length of the lining 516. In particular, the length of lining 516 may be wound about the proximal ring 512a. Rotation of the proximal ring 512a in direction P, as shown in Fig. 8, increases the length of the lining 516 that is wound about the proximal ring 512. Rotation of the proximal ring 512a in a direction opposite to that of arrow P decreases the length of the lining 516 wound about the proximal ring 512a. Accordingly, the overall length L of the intermediate section 517 may be adjusted by rotation of the proximal ring 512a.

Each of the proximal and distal rings 512a, 514a may be formed from a compressible and resilient material that is transitionable between a first condition having a first diameter and a second condition having a second diameter. The liner 516, which forms the intermediate section 517, may be a thin, pliable sheet or membrane. The proximal ring 512a may be rotated to facilitate adjusted the overall length of the liner member 500A and to maintain the liner 516 in a taut condition. When the liner 516 is taut, the liner 516 may facilitate maintaining the opening W within tissue T in an open position by maintaining the intermediate section 517 in a rigid or semi-rigid state.

Each of the liner members 50, 500A that are disclosed herein are configured and adapted for use with the cannula 70 and the obturator 60. In particular, each liner member 50, 500A is dimensioned to longitudinally translate through lumen 71 of the cannula 70 while in a compressed state and to be releasably coupled to the obturator 60 such that translation of the obturator 60 through the lumen 71 of the cannula 70 effects a corresponding translation of the liner member 50, 500A through the lumen 71 of cannula 70. In particular, at least one of the proximal end 12, 512 and the distal end 14, 514 of each liner 50, 500A are configured and adapted to releasably couple with at least one of the grooves 62, 64 of the obturator 60 such that the liner member 50, 500A may be compressed between the obturator 60 and the lumen 71 of the cannula 70.

The use and operation of the opening protection device 100 will now be described with reference to Figs. 5-7. While the use and operation of the opening protection device 100 will be described with reference to the liner member 50, the use and operation of the opening protection device 100 is substantially similar when used with liner member 500A.

As shown in Fig. 5, in a first condition, the opening protection device 100 has the liner member 50 substantially or entirely positioned within the lumen 71 of the cannula 70. The liner member 50 is releasably secured to the obturator 60. In particular, flange members 12a, 14a are held between grooves 62, 64 and the inner surface of the lumen 71 of the cannula 70. The tapered nose 65 may facilitate insertion of the opening protection device 100 into the opening W of the tissue T by providing for a gradual expansion of the opening W during insertion. Once the opening protection device 100 is placed within the opening W as shown in Fig 5, the next step is to advance the obturator 60 distally through the lumen 71 of the cannula 70, thereby advancing the liner member 50 out from within the lumen 71 of the cannula 70.

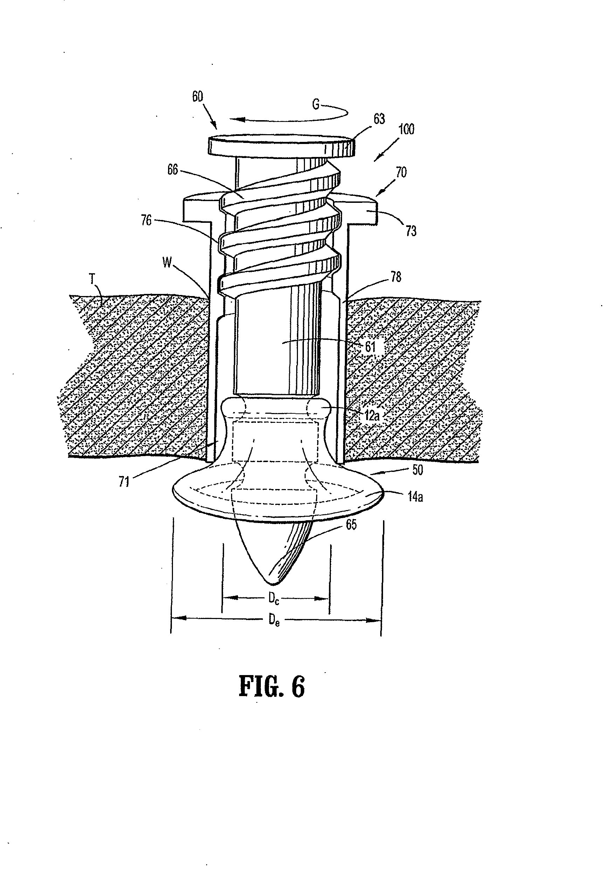

As shown in Fig. 6, advancement of the liner member 50 out from the lumen 71 of the cannula 70, the distal flange member 14a is no longer bounded by the inner surface of the lumen 71 of the cannula 70. The distal translation and advancement of the obturator 60 through the cannula may be effected by rotating the obturator 60 in direction G (Fig. 6). The corresponding threading 66 of the obturator 60 and the threading 76 of the cannula 70 are configured and adapted such that radial translation of the obturator 60 in direction G effects relative distal axial translation of the obturator 60 with respect to the cannula 70. Conversely, radial translation of the obturator in a direction opposite directional arrow G would effect a proximal axial translation of the obturator 60 with respect to the cannula 70.

As described above, the cannula 70 and the obturator 60 are shown as having corresponding threading 76, 66, respectively, such that relative rotation of the cannula 70 and the obturator 60 effects deployment of the liner member 50. However, deployment of the liner member 50 may be effected through other means. For example, the obturator 60 may be axially translated, not rotated, through the lumen 71 of the cannula 70 by applying a sufficient force to overcome any frictional resistance between the liner member 50 and the surfaces of the lumen 71. In an embodiment, the obturator 60 and/or the surfaces of the lumen 71 in contact with the obturator 60 may be formed from or include a material, e.g., a textured or rubberized surface, that would provide frictional resistance to the relative movement of the obturator 60 and the cannula 70 to facilitate a controlled advancement of the obturator 60 through the lumen 71 of the cannula 70.

As the liner member 50 is translated out from the lumen 71 of the cannula 70, the biasing force of the distal flange member 14a effects transition of the distal flange member 14a from compressed diameter Dc to expanded diameter De. The expanded diameter De of the distal flange member 14a facilitates anchoring of the liner member 50 within the opening W since the opening W has a lesser dimension than the expanded diameter De. The proximal flange member 12a is positioned at the proximal surface of the opening W and the obturator 60 and the cannula 70 are removed from the opening W while leaving the liner member 50 anchored within the opening W, as shown in Fig. 7. Anchoring of the liner member 50 is further facilitated by the biasing force of the proximal flange member 12a. In particular, the biasing force of the proximal flange member 12a transitions the radial dimension of the proximal flange member 12a from compressed diameter Dc to expanded diameter De.

Each of the embodiments described above are provided for illustrative purposes only. It will be understood that various modifications may be made to the embodiments of the present disclosure. Therefore, the above description should not be construed as limiting, but merely as exemplifications of embodiments. Those skilled in the art will envision other modifications within the scope and spirit of the present disclosure.

The invention may be described by reference to the following numbered paragraphs:
1. An opening protection device, comprising:
   a liner member, the liner member transitionable between a compressed condition and an expanded condition, the liner member defining a first width in the compressed condition and a second width in the expanded condition, the second width greater than the first width, the liner member biased toward the expanded condition; and
   a cannula including a longitudinally extending lumen, the liner member translatable through the lumen.
2. The opening protection device of paragraph 1, further comprising an obturator configured and adapted to engage the liner member to facilitate translation of the liner member through the cannula.
3. The opening protection device of paragraph of paragraph 2, wherein the obturator includes threading longitudinally extending along an outer surface thereof, and cannula includes threading longitudinally extending along an inner surface thereof, the threading of the obturator corresponding with the threading of the cannula.
4. The opening protection device of paragraph 3, wherein rotational movement of the obturator relative to the cannula effects translation of the obturator through the lumen of the cannula.
5. The opening protection device of paragraph 1, wherein the liner member comprises a proximal flange, a distal flange, and a lining between the proximal and distal flanges, the proximal and distal flanges transitionable between compressed and expanded conditions, the proximal and distal flanges defining a first diameter in the compressed condition and a second diameter in the expanded condition, the second diameter greater than the first diameter.
6. The opening protection device of paragraph 5, wherein the obturator includes proximal and distal grooves, the proximal and distal grooves engageable with the proximal and distal flanges.
7. A method for deploying an opening protection device into a bodily opening, comprising:
   providing:
      a tubular liner member defining a radial dimension, the radial dimension transitionable between a first diameter and a second diameter, the second diameter greater than the first diameter, the liner member biased toward the second diameter; and
      a cannula defining a longitudinally extending lumen, the lumen defining a diameter, the liner member translatable through the lumen, the liner member defining the first diameter while within the lumen, the first diameter corresponding to the diameter of the lumen, the liner member transitioning toward the second diameter in response to ejection from the cannula; and
      placing the cannula within a bodily opening, the cannula including the liner member positioning within the cannula;
      distally translating the liner member through the lumen of the cannula; and proximally translating the cannula out from the opening.

## Claims

1. An opening protection device, comprising:
a liner member, the liner member transitionable between a compressed condition and an expanded condition, the liner member defining a first width in the compressed condition and a second width in the expanded condition, the second width greater than the first width, the liner member biased toward the expanded condition; and
a cannula including a longitudinally extending lumen, the liner member translatable through the lumen.

2. The opening protection device of claim 1, further comprising an obturator configured and adapted to engage the liner member to facilitate translation of the liner member through the cannula.

3. The opening protection device of claim 2, wherein the obturator includes threading longitudinally extending along an outer surface thereof, and cannula includes threading longitudinally extending along an inner surface thereof, the threading of the obturator corresponding with the threading of the cannula.

4. The opening protection device of claim 3, wherein rotational movement of the obturator relative to the cannula effects translation of the obturator through the lumen of the cannula.

5. The opening protection device of any preceding claim, wherein the liner member comprises a proximal flange, a distal flange, and a lining between the proximal and distal flanges, the proximal and distal flanges transitionable between compressed and expanded conditions, the proximal and distal flanges defining a first diameter in the compressed condition and a second diameter in the expanded condition, the second diameter greater than the first diameter.

6. The opening protection device of claim 5, wherein the obturator includes proximal and distal grooves, the proximal and distal grooves engageable with the proximal and distal flanges.

7. A method for deploying an opening protection device into a bodily opening, comprising:
providing:
a tubular liner member defining a radial dimension, the radial dimension transitionable between a first diameter and a second diameter, the second diameter greater than the first diameter, the liner member biased toward the second diameter; and
a cannula defining a longitudinally extending lumen, the lumen defining a diameter, the liner member translatable through the lumen, the liner member defining the first diameter while within the lumen, the first diameter corresponding to the diameter of the lumen, the liner member transitioning toward the second diameter in response to ejection from the cannula; and
placing the cannula within a bodily opening, the cannula including the liner member positioning within the cannula;
distally translating the liner member through the lumen of the cannula; and
proximally translating the cannula out from the opening.
